(19) Europäisches Patentamt / European Patent Office / Office européen des brevets

(11) **EP 3 901 182 A1**

(12) **EUROPEAN PATENT APPLICATION**
published in accordance with Art. 153(4) EPC

(43) Date of publication:
**27.10.2021 Bulletin 2021/43**

(21) Application number: **19900702.2**

(22) Date of filing: **02.12.2019**

(51) Int Cl.:
$C08F\ 6/24$ (2006.01)   $C08F\ 220/06$ (2006.01)
$C08F\ 220/28$ (2006.01)   $C08F\ 2/32$ (2006.01)
$B01J\ 20/26$ (2006.01)   $B01J\ 20/28$ (2006.01)
$B01J\ 20/30$ (2006.01)

(86) International application number:
**PCT/JP2019/047068**

(87) International publication number:
**WO 2020/129594 (25.06.2020 Gazette 2020/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.12.2018 JP 2018235785**
**17.12.2018 JP 2018235789**

(71) Applicant: **Sumitomo Seika Chemicals Co., Ltd.**
**Kako-gun, Hyogo 675-0145 (JP)**

(72) Inventors:
• **UESAKA Shohei**
**Himeji-shi, Hyogo 672-8076 (JP)**
• **YOKOYAMA Keiichi**
**Himeji-shi, Hyogo 672-8076 (JP)**

(74) Representative: **Nederlandsch Octrooibureau**
**P.O. Box 29720**
**2502 LS The Hague (NL)**

(54) **WATER ABSORBENT RESIN, ABSORBENT BODY, ABSORBENT ARTICLE, AND PRODUCTION METHOD FOR WATER ABSORBENT RESIN**

(57)   A water-absorbent resin (21) has a shape in which a plurality of particles (40) having a substantially spherical shape is connected in a chain shape.

Fig.2

## Description

### Technical Field

The present invention relates to a water-absorbent resin, an absorbent, an absorbent article, and a method for producing a water-absorbent resin.

### Background Art

Conventionally, absorbents containing a water-absorbent resin and hydrophilic fibers (for example, pulp) have been used for sanitary products such as paper diapers. Since sanitary products are required to be comfortable even when being worn for a long period of time, studies are underway to reduce the size of absorbents and improve absorption performances of absorbents. For example, Patent Literature 1 provides sanitary products that are less likely to cause rashes even when being used for a long period of time by improving a water absorption performance of a water-absorbent resin contained in an absorbent.

### Citation List

### Patent Literature

[Patent Literature 1] Japanese Unexamined Patent Publication No. 2009-84472

### Summary of Invention

### Technical Problem

According to the findings of the inventors of the present invention, it is effective to reduce the content of hydrophilic fibers, which are bulkier than a water-absorbent resin, to reduce the thickness of an absorbent. However, when the content of hydrophilic fibers having a function of diffusing a liquid in the absorbent is reduced, localization of liquid absorption occurs in the absorbent, and as a result, the absorption capacity of the absorbent may not be sufficiently exhibited in some cases.

With the foregoing in view, it is an object of the present disclosure to provide a water-absorbent resin, an absorbent containing the water-absorbent resin, and a method for producing the water-absorbent resin, where the water-absorbent resin enables configuration of a thin absorbent, enables a reduction in amount of reversion from the absorbent, and can impart sufficient diffusion power to the absorbent.

### Solution to Problem

The inventors of the present invention conducted research after they thought that, when it is possible to produce a new water-absorbent resin having functions of both a conventional water-absorbent resin and hydrophilic fibers, it is possible to improve a water absorption performance of an absorbent or maintain them in required and sufficient states while making the absorbent thinner, as compared to those of a conventional absorbent. As a result, they have found that, in a case where a water-absorbent resin has a specific shape, an absorbent can be made thinner, an amount of reversion of the absorbent can be reduced, and a liquid can be sufficiently diffused in the absorbent.

That is, a water-absorbent resin according to one aspect of the present disclosure has a shape in which a plurality of particles having a substantially spherical shape is connected in a chain shape.

In the water-absorbent resin having the shape in which a plurality of particles having a substantially spherical shape is connected in a chain shape, the particles have water absorption performances as characteristics of the water-absorbent resin, and enable sufficient diffusion of a liquid due to the unique shape of them.

Furthermore, it is possible to adopt an aspect in which the water-absorbent resin is a copolymer of two or more monomers.

Furthermore, an absorbent according to one aspect of the present disclosure contains the above-described water-absorbent resin.

The above-described absorbent contains the water-absorbent resin having the shape in which a plurality of particles having a substantially spherical shape is connected in a chain shape. Accordingly, the absorbent also has an absorption performance and can sufficiently diffuse a liquid.

Furthermore, the inventors of the present invention have found that, in a case where a water-absorbent resin used for an absorbent has a specific shape, a sensation of the skin becomes pleasant when the absorbent is formed by combining this water-absorbent resin with hydrophilic fibers. That is, the inventors of the present invention have found that a thin absorbent can be configured, and not only an amount of reversion of the absorbent can be reduced, but also an effect of improving a sensation of the skin can be obtained.

That is, an absorbent according to one aspect of the present disclosure contains a water-absorbent resin having a shape in which a plurality of particles having a substantially spherical shape is connected in a chain shape, and hydrophilic fibers.

Because the water-absorbent resin having a shape in which a plurality of particles having a substantially spherical shape is connected in a chain shape is softer than a conventional water-absorbent resin, a sensation of the skin becomes favorable according to the absorbent formed using this water-absorbent resin. In the present disclosure, the sentence "a sensation of the skin is pleasant (is favorable)" refers to a state in which both deviation of friction coefficient (MMD) and surface roughness (SMD), which represent friction characteristics, show small values, and a state in which a smooth tactile sensation can be obtained.

Furthermore, the water-absorbent resin may be a copolymer of two or more monomers.

Furthermore, an absorbent article according to one aspect of the present disclosure contains the above-described absorbent.

The above-described absorbent article contains the absorbent containing the above-described water-absorbent resin having the shape in which a plurality of particles having a substantially spherical shape is connected in a chain shape. Accordingly, a sensation of the skin becomes favorable also according to the absorbent article.

Furthermore, a method for producing a water-absorbent resin according to one embodiment of the present disclosure includes: copolymerizing a monomer A and a monomer B by a reverse phase suspension polymerization method; and drying a hydrogel obtained in the copolymerizing, in which the monomer A contains at least one of (meth)acrylic acid and a neutralized product thereof, the monomer B contains alkoxypolyalkylene glycol (meth)acrylate, and in the copolymerizing, a ratio ($\alpha/\beta$) of a molar amount ($\alpha$) of the monomer A to be provided for the copolymerization to a molar amount ($\beta$) of the monomer B to be provided for the copolymerization is 1/4 to 4.

According to the above-described method for producing a water-absorbent resin, it is possible to produce a water-absorbent resin which has a high water absorption performance and can sufficiently diffuse a liquid, where the water-absorbent resin has a shape in which a plurality of particles having a substantially spherical shape is connected in a chain shape. In the present specification, "acrylic" and "methacrylic" are collectively referred to as "(meth)acrylic."

It is possible to adopt an aspect in which, in the copolymerizing, at least one of sucrose fatty acid ester and polyglycerin fatty acid ester is added to a reaction system as a surfactant.

As described above, by adding at least one of sucrose fatty acid ester and polyglycerin fatty acid ester as a surfactant in the copolymerizing, it is possible to suitably produce a water-absorbent resin having a shape in which a plurality of particles having a substantially spherical shape is connected in a chain shape.

Furthermore, it is possible to adopt an aspect in which, in the copolymerizing, polymerization is performed in the presence of a polymer-based dispersant.

As described above, by carrying out the polymerization in the presence of a polymer-based dispersant, particles are appropriately dispersed in the copolymerizing and are unlikely to be agglomerated. Thereby, it is possible to suitably obtain a water-absorbent resin having a shape in which a plurality of particles having a substantially spherical shape is connected in a chain shape.

It is possible to adopt an aspect in which, in the drying, the hydrogel is dehydrated by azeotropic distillation of a hydrocarbon dispersion medium until a water content reaches 50% by mass or less.

As described above, in the drying, by dehydrating the hydrogel by azeotropic distillation of a hydrocarbon dispersion medium until a water content reaches 50% by mass or less, it is possible to suitably produce a water-absorbent resin having a shape in which a plurality of particles having a substantially spherical shape is connected in a chain shape.

According to the present disclosure, a water-absorbent resin, an absorbent containing the water-absorbent resin, and a method for producing the water-absorbent resin are provided, where the water-absorbent resin enables configuration of a thin absorbent, enables a reduction in amount of reversion from the absorbent, and enables sufficient diffusion of a liquid within the absorbent.

**Advantageous Effects of Invention**

According to the present invention, an absorbent providing a pleasant sensation of the skin, and an absorbent article containing the absorbent are provided.

**Brief Description of Drawings**

Fig. 1 is a cross-sectional view of an absorbent article containing an absorbent according to one embodiment of the present disclosure.

Fig. 2(A) and Fig. 2(B) are views showing examples of a water-absorbent resin according to one embodiment of the present invention.

Fig. 3(A) to Fig. 3(F) are photographs showing SEM images of one water-absorbent resin according to Examples.

Fig. 4 is a photograph showing an SEM image of a plurality of water-absorbent resins according to Examples.

Fig. 5 is a view showing a measurement device used for measuring a water-absorbing ability of a water-absorbent resin under pressure.

**Description of Embodiments**

Hereinafter, embodiments for carrying out the present disclosure will be described in detail with reference to the accompanying drawings. In describing the drawings, the same elements are designated by the same reference numerals, and duplicate descriptions will be omitted. In the present specification, in a case where a plurality of lower limit values and a plurality of upper limit values are separately described, an arbitrary lower limit value and an arbitrary upper limit value can be selected to obtain a specific numerical value range. Furthermore, values disclosed in Examples of the present specification or numerical values that can be unambiguously derived from Examples can be used as candidates for lower limit values or upper limit values. Furthermore, each component disclosed in the present specification can be appropriately combined.

(Absorbent article and absorbent)

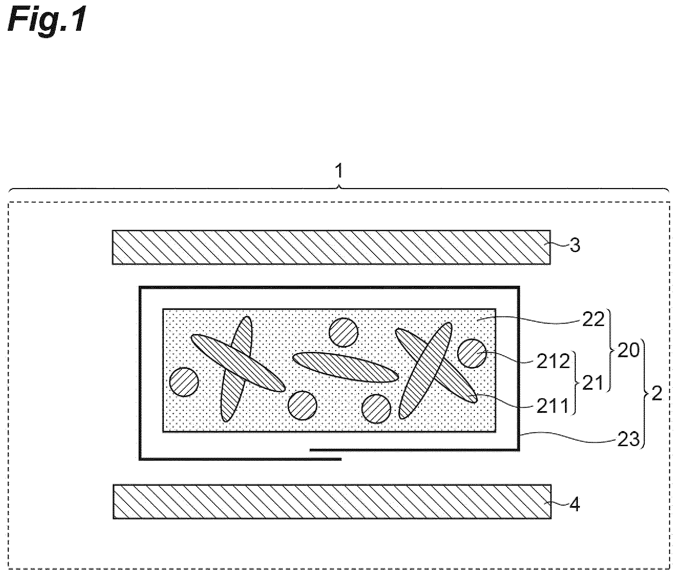

Fig. 1 is a cross-sectional view showing a configuration of an absorbent article containing an absorbent according to one embodiment of the present disclosure. An absorbent article 1 shown in Fig. 1 includes an absorbent 2, a liquid permeable sheet 3, and a liquid impermeable sheet 4. The absorbent 2 is sandwiched between the liquid permeable sheet 3 and the liquid impermeable sheet 4 which are disposed to face each other.

Examples of the absorbent article include paper diapers, incontinence pads, sanitary napkins, incontinence liners, panty liners, pet sheets, drip sheets for foods, and the like. In consideration of realization of a thin absorbent by a water-absorbent resin of the present disclosure, and products in which a sensation of the skin is improved, the absorbent article is preferably a product that comes into contact with the body (paper diapers, incontinence pads, sanitary napkins, incontinence liners, panty liners, and the like). By using the water-absorbent resin of the present disclosure, the absorbent and the absorbent article containing the absorbent can be made thinner, and thereby it is possible to obtain the absorbent article that is inconspicuous even when being worn and enables reduction in transportation costs. By using the absorbent of the present disclosure, it is possible to improve a sensation of the skin from the absorbent article containing the absorbent. In a case where the absorbent article 1 is used in a product that comes into contact with the body, the liquid permeable sheet 3 is disposed on a side that comes into contact with the body, and the liquid impermeable sheet 4 is disposed on the side opposite thereto.

The absorbent 2 includes at least an absorption core 20. In the present embodiment, the absorbent 2 further includes a core wrap 23 in addition to the absorption core 20. The absorption core 20 is a core member that influences an absorption performance of the absorbent 2, and includes at least a water-absorbent resin 21. In the present embodiment, the absorption core 20 further includes hydrophilic fibers 22 in addition to the water-absorbent resin 21, but the absorption core 20 may not include the hydrophilic fibers 22. The core wrap 23 is a sheet-like member that retains the shape of the absorption core 20. Examples of the core wrap 23 include tissues, non-woven fabrics, and the like. In a case where the core wrap 23 is used, a shape retention method for the absorption core 20 is not particularly limited. For example, the shape of the absorption core 20 may be retained by wrapping the absorption core 20 with one core wrap 23 as shown in Fig. 1, or the shape of the absorption core 20 may be retained by being sandwiched between two core wraps 23 (not shown).

Although there are gaps between the liquid permeable sheet 3, the absorbent 2, and the liquid impermeable sheet 4 as shown in Fig. 1 to make it easy to understand the layer configuration of the absorbent article 1, but originally, these components are in close contact with each other (the same applies to the core wrap 23 and the absorption core 20 of the absorbent 2).

The hydrophilic fibers 22 are members having a function of diffusing a liquid inside the absorbent 2 by a capillary phenomenon. Examples of the hydrophilic fibers 22 include cellulose fibers, fibers made of synthetic resin, and the like. Examples of the cellulose fibers include natural cellulose fibers such as cotton-like pulp, mechanical pulp, chemical pulp, and semi-chemical pulp which are obtained from wood; regenerated cellulose fibers such as rayon and fibril rayon; semi-synthetic cellulose fibers such as acetate and triacetate; and the like. Examples of the fibers made of synthetic resin include fibers made of hydrophilized polyamide, polyester, polyolefin, and the like. These fibers may be used alone or as a mixed fiber in which two or more kinds thereof are combined.

A use amount of the hydrophilic fibers 22 in the absorbent 2 is, for example, 60% by mass or less, preferably 55% by mass or less, and more preferably 50% by mass or less, based on a mass of the absorption core 20 (100% by mass) to realize thinning of the absorbent 2 and the absorbent article 1. A use amount of the hydrophilic fibers 22 may be 0% by mass (that is, the absorption core 20 may not contain the hydrophilic fibers 22). Among them, when the absorption core 20 contains a small amount of the hydrophilic fibers 22, liquid diffusion power of the absorbent 2 and the absorbent article 1 of the present invention can be reinforced. Accordingly, a use amount of the hydrophilic fibers 22 is preferably 10% by mass or more, more preferably 20% by mass or more, and even more preferably 30% by mass or more, based on a mass of the absorption core 20.

The absorption core 20 may further contain synthetic fibers made of synthetic resins such as polyamide, polyester, and polyolefin as a reinforcing agent. Furthermore, adhesive binders such as heat-sealing synthetic fibers, hot melt adhesives, and adhesive emulsions may be added to the absorption core 20 to enhance morphological retention of the absorbent.

Furthermore, a thickness of the absorbent 2 can be, for example, 0.1 to 10 mm. Examples of structures of the absorption core 20 in the absorbent 2 include a mixing structure in which the water-absorbent resin 21 and the hydrophilic fibers 22 are uniformly blended; a sandwich structure in which the water-absorbent resin 21 is held between a plurality of layers of the hydrophilic fibers 22; and the like. However, the absorbent 2 and the absorption core 20 of the present embodiment are not limited to such examples.

A use amount of the water-absorbent resin 21 in the absorbent 2 is, for example, 5% to 100% by mass, 30% to 90% by mass, or 50% to 80% by mass, based on a mass of the absorption core 20 (100% by mass). When a use amount of the water-absorbent resin 21 is 5% by mass or more, an absorption capacity of the absorbent 2 becomes large, and there is a tendency that liquid leakage and reversion can be inhibited.

The water-absorbent resin 21 constituting the absorbent 2 includes a water-absorbent resin 211 (hereinafter, referred to as a chained water-absorbent resin 211) having a shape in which at least a plurality of particles having a substantially spherical shape is connected in a chain shape. The chained water-absorbent resin 211 has a long portion (that is, elongated) because particles are connected in a chain shape. The water-absorbent resin 21 may include a water-absorbent resin 212 (hereinafter, referred to as a non-chain water-absorbent resin 212) having a shape different from that of the chained water-absorbent resin 211. In the present embodiment, as shown in Fig. 1, the water-absorbent resin 21 includes the chained water-absorbent resin 211 and the non-chain water-absorbent resin 212. Details of the chained water-absorbent resin 211 will be described later.

The non-chain water-absorbent resin 212 is a resin that does not correspond to the chained water-absorbent resin 211 to be described later, and is a water-absorbent resin having, for example, a substantially spherical shape, a crushed shape, a granular shape, or a shape in which a plurality of particles thereof is agglomerated. The shape of the non-chain water-absorbent resin 212 is not particularly limited as long as it is different from that of the chained water-absorbent resin 211. Furthermore, a lower limit value of a proportion of the chained water-absorbent resin 211 in the water-absorbent resin 21 contained in the absorbent 2 is preferably 20% by mass, is more preferably 25% by mass, and is even more preferably 40% by mass, based on a total amount of the water-absorbent resin 21 (100% by mass). Furthermore, an upper limit value thereof is, for example, 50% by mass, preferably 60% by mass, more preferably 70% by mass, and particularly preferably 90% by mass. An upper limit value may be 100% by mass (that is, only the chained water-absorbent resin 211 is used as the water-absorbent resin 21), but when a proportion of the chained water-absorbent resin 211 is set within the above-mentioned range, the absorbent can be made thinner, an amount of reversion of the absorbent can be further reduced, and diffusibility of a liquid can be more imparted to the absorbent.

Examples of the liquid permeable sheet 3 include non-woven fabrics made of synthetic resin such as polyethylene, polypropylene, polyester, and polyamide, and porous synthetic resin sheets. Furthermore, examples of the liquid impermeable sheet 4 include sheets made of synthetic resins such as polyethylene, polypropylene, and polyvinyl chloride, and sheets made of a composite material of these synthetic resins and a non-woven fabric. A sizes and a thickness of the liquid permeable sheet 3 and the liquid impermeable sheet 4 are appropriately adjusted depending on usage applications of the absorbent article, and the like. Since a thin absorbent can be obtained by using the chained water-absorbent resin 211 of the present invention, the thin absorbent article 1 can be obtained even when a relatively thick liquid permeable sheet 3 and/or liquid impermeable sheet 4 is used.

The absorbent 2 and the absorbent article 1 may further contain additives such as lubricants, deodorants, antibacterial agents, and fragrances. An addition site of these additives can be appropriately set based on the function of an additive. For example, an additive can be added within the absorption core 20, between the core wrap 23 and the absorption core 20, or between the liquid permeable sheet 3 and/or the liquid impermeable sheet 4 and the core wrap 23.

In the absorbent 2, a deviation of friction coefficient (MMD) to be described later is preferably more than 0 and 0.03 or less, and is more preferably more than 0 and 0.01 or less. The deviation of friction coefficient (MMD) is an index of roughness, and indicates more roughness as a value becomes larger. Therefore, when a measured value of a deviation of friction coefficient (MMD) becomes smaller, there is a roughness sensation becomes less, which is preferable for use in an absorbent. A method for measuring a deviation of friction coefficient (MMD) is as described in Examples of the present specification.

A surface roughness (SMD) of the absorbent 2 is preferably more than 0 and 5.0 or less, and is more preferably more than 0 and 3.0 or less. The surface roughness (SMD) is an index related to unevenness of a surface, and indicates that unevenness of a surface is severe as a value becomes larger. Therefore, when a measured value of a surface roughness (SMD) becomes smaller, there is an unevenness sensation becomes less, which is preferable for use in an absorbent. A method for measuring a surface roughness (SMD) is as described in Examples of the present specification.

The above-mentioned deviation of friction coefficient (MMD) and surface roughness (SMD) are both indices related to a sensation of the skin. The absorbent 2 in which both the deviation of friction coefficient (MMD) and the surface roughness (SMD) satisfy the above-described ranges has a favorable sensation of the skin.

(Water-absorbent resin)

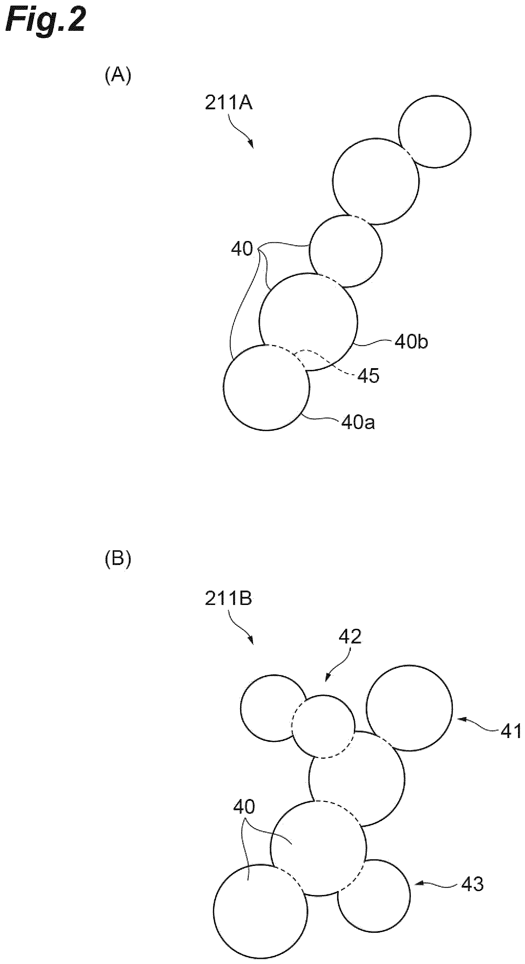

The chained water-absorbent resin 211 contained in the water-absorbent resin 21 according to the present embodiment will be described. Fig. 2 shows an example of the chained water-absorbent resin 211. As shown in Fig. 2, the chained water-absorbent resin 211 has a shape in which a plurality of particles 40 having a substantially spherical shape is connected in a chain shape.

The particles 40 having a substantially spherical shape are particles in which a cross-section is a substantially circular. A cross-sectional shape of the particles 40 is not limited to a perfect circle, and it may be an ellipse or may be an irregular shape close to a circle. For example, a shape in which an aspect ratio is 1.5 or less can be called "substantially spherical shape." Furthermore, a particle size of the particles 40 is, for example, 10 $\mu$m to 600 $\mu$m, and is preferably 50 $\mu$m to 500 $\mu$m. A particle size of the particles 40 can be controlled by, for example, a use amount of a surfactant to be described later, a stirring speed during polymerization, and the like.

Furthermore, the plurality of particles 40 is connected in a state in which each of the particles can be distinguished. For example, a particle 40a and a particle 40b, which are contained in the plurality of particles 40 in the chained water-absorbent resin 211A of Fig. 2(A), are disposed adjacent to each other, and the particle 40a and the particle 40b are connected so that a part of the surface of the particle 40a overlaps a part of the surface of the particle 40b. A width (width when a connecting portion is viewed from the side) of a boundary 45, which is between the particle 40a and the particle 40b, at the connecting portion of the particle 40a and the particle 40b is shorter than a maximum size of the particle 40a and the particle 40b. Accordingly, a constricted boundary 45 can be specified between the adjacent particles 40a and 40b, and the particle 40a and the particle 40b can be distinguished from each other with this boundary 45 therebetween as a boundary. In this manner, the plurality of particles 40 is connected in a state in which adjacent particles can be distinguished from each other. By linearly connecting the plurality of particles 40 having a substantially spherical shape in a state in which they can be distinguished from each other, it is possible to obtain the chained water-absorbent resin 211 having a shape in which the "particles are connected in a chain shape." This chained water-absorbent resin 211 has a shape as if were beads (a tool in which a plurality of beads having through holes is continuously connected through a thread so that a shape is linear and there is no gap between adjacent beads).

Furthermore, when the plurality of particles 40 is connected in a chain shape, it means a state in which the plurality of particles 40 is connected in a linear or branched chain shape. For example, in the chained water-absorbent resin 211, at least five or more particles 40 are connected in a chain shape, and among them, three or more particles 40 form a main chain. The main chain corresponds to the longest portion of the chained water-absorbent resin 211. In the chained water-absorbent resin 211A shown in Fig. 2(A), five particles 40 form a straight line. Furthermore, in the chained water-absorbent resin 211B shown in Fig. 2(B), seven particles 40 are connected in a chain shape, and among them, four particles 40 form a main chain 41, and a branched chain 42 composed of two particles 40 and a branched chain 43 composed of one particle 40 are connected to a main chain 41. As described above, the structure of the chained water-absorbent resin 211 is not particularly limited as long as the plurality of particles 40 is connected in a chain shape. Furthermore, the size of each of the branched particles 40 is not particularly limited, and for example, particles 40 having different particle sizes may be connected to each other. The number of particles 40 connected in a chain shape is preferably 5 or more, and is more preferably 10 or more. Furthermore, an upper limit value thereof is not particularly limited, and a longer chained water-absorbent resin can be obtained as a number becomes larger, but in reality, the number is 100 or less.

It is most desirable that the chained water-absorbent resin 211 is composed of only the particles 40 having a substantially spherical shape (that is, a configuration having only a "chained portion" in which the particles 40 having a substantially spherical shape are continuously connected), but the particles 40 having a substantially spherical shape may be intermittently connected via particles that are not substantially spherical. That is, non-spherical particles may be interposed between adjacent particles 40 having a substantially spherical shape. Furthermore, the chained water-absorbent resin 211 may have a structure in which non-spherical particles are connected to the end of the chained portion. In a case where the chained water-absorbent resin 211 contains particles that are not substantially spherical, a proportion of the particles 40 having a substantially spherical shape is 70% by mass or more, is preferably 80% by mass or more, and is more preferably 90% by mass or more.

The chained water-absorbent resin 211 in which the plurality of particles 40 having a substantially spherical shape is connected in a chain shape has a long shape in which an extending direction of the main chain is a longitudinal direction.

The particle size of the chained water-absorbent resin 211 is, for example, 150 $\mu$m, is preferably 200 $\mu$m, and is more preferably 250 $\mu$m. Furthermore, an upper limit value thereof is, for example, 5,000 $\mu$m, is preferably 3,000 $\mu$m, is more preferably 2,500 $\mu$m, and is even more preferably 2,000 $\mu$m.

A sieve is used to measure the particle size of the chained water-absorbent resin 211. Specifically, JIS standard sieves were combined in the following order from the top: a sieve having an opening of 850 $\mu$m, a sieve having an opening of 600 $\mu$m, a sieve having an opening of 500 $\mu$m, a sieve having an opening of 425 $\mu$m, a sieve having an opening of 300 $\mu$m, a sieve having an opening of 250 $\mu$m, a sieve having an opening of 150 $\mu$m, and a saucer. Thereafter, the chained water-absorbent resin 211 is fed to the topmost sieve among the combination of the sieves, shaken for 20 minutes using a Ro-Tap shaker, and thereby classified. After the classification, a mass of the chained water-absorbent resin remaining on each of the sieves is calculated as a mass percentage with respect to a total amount to determine a particle size distribution. By integrating values on the sieves in descending order of the particle sizes with regard to the particle size distribution, a relationship between the opening of the sieve and the integrated value of mass percentages of the chained water-absorbent resin remaining on the sieve is plotted on a log-probability paper. The plotted points on the probability paper are connected with straight lines, and a particle size (median particle size) corresponding to 50% by mass of the integrated mass percentage is taken as a particle size of the chained water-absorbent resin 211.

Since the chained water-absorbent resin 211 itself is a water-absorbent resin, it has a higher water retention amount than hydrophilic fibers and can reduce an amount of reversion from the absorbent. Furthermore, the chained water-absorbent resin 211 is softer than a conventional water-absorbent resin (non-chain water-absorbent resin), and therefore can constitute an absorbent and an absorbent article which have a favorable sensation of the skin. Furthermore, the chained water-absorbent resin 211 can sufficiently diffuse a liquid in the absorbent. Furthermore, the chained water-absorbent resin 211 is less bulky than the hydrophilic fibers, and therefore can constitute the thin absorbent 2.

A lower limit value of a water retention amount of the chained water-absorbent resin 211 for physiological saline is, for example, 15 g, is preferably 20 g, and is more preferably 25 g, with respect to 1 g of the chained water-absorbent resin. Furthermore, an upper limit value thereof is, for example, 60 g, is preferably 50 g, and is more preferably 45 g. A method for measuring a water retention amount for physiological saline is as described in Examples of the present specification.

A lower limit value of a water absorption rate of the chained water-absorbent resin 211 for physiological saline is, for example, 10 seconds, is preferably 20 seconds, and is more preferably 30 seconds. Furthermore, an upper limit value is, for example, 90 seconds, is preferably 70 seconds, and is more preferably 60 seconds. A method for measuring a water absorption rate is as described in Examples of the present specification.

A lower limit value of a water-absorbing ability under pressure of the chained water-absorbent resin 211 for physiological saline is, for example, 3 ml, is preferably 5 ml, and is more preferably 6 ml, with respect to 1 g of the chained water-absorbent resin. A method for measuring a water-absorbing ability under pressure is as described in Examples of the present specification.

The chained water-absorbent resin 211 exhibits a softer tactile sensation than the non-chain water-absorbent resin due to its specific shape. By using the chained water-absorbent resin 211, which has a softer tactile sensation than the non-chain water-absorbent resin, for the absorbent 2 and the absorbent article 1, an effect of improving a sensation of the skin can be obtained. In particular, in a case where a proportion of the hydrophilic fibers 22 in the absorbent 2 is low, a tactile sensation of the water-absorbent resin 21 contained in the absorbent 2 greatly affects a sensation of the skin as the absorbent 2. Accordingly, by incorporating the chained water-absorbent resin 211 having a soft tactile sensation in the absorbent 2, a sensation of the skin as the absorbent 2 becomes favorable. Furthermore, since the absorbent 2 has a favorable sensation of the skin, a sensation of the skin as the absorbent article 1 also becomes favorable.

(Method for producing chained water-absorbent resin)

An example of a method for producing the chained water-absorbent resin 211 will be described. The chained water-absorbent resin 211 can be produced, for example, by copolymerizing two or more kinds of monomers. Specifically, the method for producing the chained water-absorbent resin 211 includes a polymerization step of copolymerizing a monomer A and a monomer B (of the type different from that of the monomer A) by a reverse phase suspension polymerization method; and a drying step of drying a hydrogel obtained in the polymerization step.

<Polymerization step>

In the present embodiment, the monomer A used in the polymerization step contains at least one of (meth)acrylic acid and a neutralized product thereof, and the monomer B contains alkoxypolyalkylene glycol (meth)acrylate. In the present specification, "acrylic" and "methacrylic" are collectively referred to as "(meth)acrylic."

The neutralized product of (meth)acrylic acid can be obtained by, for example, neutralizing (meth)acrylic acid with an alkaline neutralizing agent.

From the viewpoint of increasing an osmotic pressure of the chained water-absorbent resin, increasing a water absorption rate, and further improving water absorption properties, a neutralization degree of the neutralized product is 10 to 100 mol% of an acidic group in (meth)acrylic acid, is preferably 50 to 90 mol% thereof, and is more preferably 60 to 80 mol% thereof.

Examples of alkaline neutralizing agents include alkali metal salts such as sodium hydroxide, sodium carbonate, sodium hydrogen carbonate, potassium hydroxide, and potassium carbonate; and ammonia. These alkaline neutralizing agents may be used in a form of an aqueous solution to simplify a neutralizing operation. The above-mentioned alkaline neutralizing agents may be used alone or in combination of two or more kinds thereof. Neutralization of the acid group of (meth)acrylic acid can be performed by, for example, dropwise adding an aqueous solution (neutralizing aqueous solution) containing an alkaline neutralizing agent such as sodium hydroxide or potassium hydroxide to an aqueous solution of (meth)acrylic acid, and mixing them. A concentration of the neutralizing aqueous solution is, for example, 20% by mass to 50% by mass.

The neutralized product of (meth)acrylic acid is an alkali metal salt of (meth)acrylic acid partially neutralized at the above-mentioned degree of neutralization, and is more preferably a sodium salt of (meth)acrylic acid neutralized at the above-mentioned degree of neutralization.

The alkoxypolyalkylene glycol (meth)acrylate contained in the monomer B is represented by General Formula (1).

$$CH_2 = CHCO(OCH_2CH_2)_nOR \qquad (1)$$

In General Formula (1) above, "n" represents the number of repeating units of ethylene glycol, and is an integer larger than 0. A lower limit value of n is, for example, 5, is preferably 7, and is more preferably 9. An upper limit value of n is, for example, 18, is preferably 15, and is more preferably 13. In General Formula (1) above, "R" represents hydrogen or a substituted or unsubstituted alkyl group. Because it is easy to copolymerize with the above (meth)acrylic acid or a neutralized product thereof, the number of carbon atoms of R is preferably 4 or less, is more preferably 3 or less, is even more preferably 2 or less, and is most preferably 1. R is preferably an unsubstituted alkyl group having 3 or less carbon atoms (a methyl group, an ethyl group, a propyl group, or an isopropyl group), and is more preferably an unsubstituted alkyl group having 2 or less carbon atoms (a methyl group or an ethyl group), and is particularly preferably an unsubstituted alkyl group having 1 carbon number (a methyl group).

A lower limit value of a ratio $(\alpha/\beta)$ of a molar amount $(\alpha)$ of the monomer A to be provided for the copolymerization to a molar amount $(\beta)$ of the monomer B to be provided for the copolymerization is preferably 1/4, is more preferably 3/7, and is even more preferably 2/3. An upper limit value is preferably 4, is more preferably 7/3, and is even more preferably 3/2. When a ratio $(\alpha/\beta)$ of these monomers is within the above range, because the particles 40 having a substantially spherical shape can be easily connected in a chain shape, the chained water-absorbent resin can be produced in a high yield; or because the produced chained water-absorbent resin is less likely to agglomerate, it can be mixed favorably with the hydrophilic fibers.

In the production of the chained water-absorbent resin 211, in addition to the above-mentioned two types of monomers A and B, another monomer C may be added and copolymerized. Examples of the monomer C include water-soluble ethylenically unsaturated monomers. Examples of water-soluble ethylenically unsaturated monomers include 2-(meth)acrylamide-2-methylpropanesulfonic acid and a alkali salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where a water-soluble ethylenically unsaturated monomer has an amino group, the amino group may be quaternarized.

Furthermore, as the monomer C, a water-soluble monomer other than the above-mentioned water-soluble ethylenically unsaturated monomer may be selected as long as expression of the effect of the present disclosure is not hindered. Such a monomer can be used by, for example, being mixed with an aqueous solution containing the above-mentioned water-soluble ethylenically unsaturated monomers.

In a case where the monomer C contains an acid group, the monomer C may be neutralized with an alkali neutralizing agent in the same manner as the above-mentioned neutralized product of (meth)acrylic acid. The neutralizing agent used for neutralization and a range of a preferable neutralization degree are the same as those of the neutralized product of (meth)acrylic acid.

Even in a case where the monomer C is added, a total amount of the monomer A and the monomer B is preferably 70 mol% or more, and is more preferably 80 mol% or more, with respect to a total amount of the monomers.

Furthermore, in this case, the total amount is necessarily less than 100 mol%.

In general, the monomers A to C used in the polymerization step are suitably used as an aqueous solution. In this case, a concentration of the monomers in the aqueous solution of the monomers (hereinafter referred to as a monomer aqueous solution) is generally within a range of 25% by mass to a saturated concentration, and is preferably 35% by mass to 60% by mass. Examples of water to be used include tap water, distilled water, and ion-exchanged water.

In the reverse phase suspension polymerization method, a monomer aqueous solution is dispersed in a hydrocarbon dispersion medium in the presence of a surfactant, and polymerization of the above-described monomers is performed using a water-soluble radical polymerization initiator or the like. An internal cross-linking agent may be used in the polymerization.

As the surfactant, the polymer-based dispersant, the hydrocarbon dispersion medium, water-soluble radical polymerization initiator, internal cross-linking agent, and the like used in the polymerization reaction, those that can be generally used in the reverse phase suspension polymerization method are used.

As the surfactant, among nonionic surfactants, it is possible to use sucrose fatty acid ester, polyglycerin fatty acid ester, propylene glycol fatty acid ester, sorbitan fatty acid ester, and the like. Among them, the chained water-absorbent resin can be suitably obtained by using, preferably a sucrose fatty acid ester and/or a polyglycerin fatty acid ester. An anionic surfactant may be used as the surfactant. Examples of anionic surfactants include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. Furthermore, these surfactants may be used alone or in combination of two or more kinds thereof.

An HLB (hydrophilic/lipophilic balance) of the surfactant is preferably 6 or less. A lower limit value of the HLB of the surfactant is, for example, 2, and is preferably 3. When the HLB of the surfactant is within this range, substantially spherical particles can be easily obtained, and because the particles can be easily connected to each other, a chained water-absorbent resin can be efficiently obtained.

An amount of the surfactant added to the reaction system is preferably 0.1 part by mass to 5 parts by mass, is more preferably 0.2 parts by mass to 3 parts by mass, and is even more preferably 0.5 parts by mass to 2 parts by mass, with respect to 100 parts by mass of the monomer aqueous solution to be subjected to the polymerization step, from the viewpoint that a sufficient effect is obtained within these use amounts, and these amounts are economic.

Furthermore, in the method for producing a chained water-absorbent resin of the present disclosure, it is preferable to use a polymer-based dispersant together with the surfactant for the reverse phase suspension polymerization. By using the polymer-based dispersant, particles of the water-absorbent resin are appropriately dispersed in a hydrocarbon dispersion medium, and thereby a chained water-absorbent resin can be suitably obtained without agglomeration of the particles. Examples of usable polymer-based dispersants include maleic anhydride-modified polyethylene, maleic anhydride-modified polypropylene, a maleic anhydride-modified ethylene-propylene copolymer, a maleic anhydride-modified EPDM (ethylene propylene diene terpolymer), maleic anhydride-modified polybutadiene, a maleic anhydride-ethylene copolymer, a maleic anhydride-propylene copolymer, a maleic anhydride-ethylene-propylene copolymer, a maleic anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, ethyl hydroxyethyl cellulose, and the like. Among them, from the viewpoint of dispersion stability of monomers, it is preferable to use maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, an oxidized ethylene-propylene copolymer, and the like. These polymer-based dispersants may be used alone or in combination of two or more kinds thereof.

An amount of the polymer-based dispersant added to the reaction system is preferably 0.1 part by mass to 5 parts by mass, is more preferably 0.2 parts by mass to 3 parts by mass, and is even more preferably 0.5 parts by mass to 2 parts by mass, with respect to 100 parts by mass of the monomers subjected to the polymerization step, to maintain a favorable dispersion state of the monomers in a hydrocarbon dispersion medium and obtain a dispersion effect commensurate with the use amount.

Examples of water-soluble radical polymerization initiators include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-methylpropionamidine)dihydrochloride, 2,2'-azobis[2-(N-phenylamidino)propane]dihydrochloride, 2,2'-azobis[2-(N-allylamidino)propane]dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propi onamide}, 2,2'-azobis[2-methyl-N-(2-hydroxyethyl)-propionamide], 4,4'-azobis(4-cyanovaleric acid), and the like. These radical polymerization initiators may be used alone or in combination of two or more kinds thereof.

The amount of the water-soluble radical polymerization initiator added to the reaction system is, for example, 0.005 to 1 mol with respect to 100 mol of the monomers subjected to the polymerization step. A case in which a use amount of the water-soluble radical polymerization initiator is 0.005 moles or more is efficient, because then a polymerization reaction is not required to be performed for a long period of time. When a use amount is 1 mol or less, the polymerization reaction has an appropriate speed, which is preferable.

The water-soluble radical polymerization initiator can also be used as a redox polymerization initiator when it is used in combination with a reducing agent such as sodium sulfite, sodium hydrogen sulfite, ferrous sulfate, and L-ascorbic acid.

At the time of the polymerization reaction, a chain transfer agent may be contained in the monomer aqueous solution used in the polymerization to control liquid absorption characteristics of the chained water-absorbent resin. Examples of such chain transfer agents include hypophosphites, thiols, thiolic acids, secondary alcohols, and amines.

Examples of hydrocarbon dispersion media include chained aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. These hydrocarbon dispersion media may be used alone or in combination of two or more kinds thereof. The hydrocarbon dispersion medium may include at least one compound selected from the group consisting of a chained aliphatic hydrocarbon having 6 to 8 carbon atoms and an alicyclic hydrocarbon having 6 to 8 carbon atoms. For the hydrocarbon dispersion medium, n-heptane, cyclohexane, or both n-heptane and cyclohexane may be contained, from the viewpoints that then, a state of W/O-type reverse phase suspension is favorable, and a chained water-absorbent resin having an excellent water absorption rate can be easily obtained with a suitable particle size, and industrial availability is high, and qualities are stable. Furthermore, from the same viewpoints, as a mixture of the hydrocarbon dispersion media, for example, a commercially available Exxsol Heptane (manufactured by ExxonMobil Chemical: containing n-heptane and 75% to 85% of hydrocarbons of isomers thereof) may be used.

A use amount of the hydrocarbon dispersion medium is preferably 100 to 1,000 parts by mass, is more preferably 150 to 800 parts by mass, and is even more preferably 200 to 700 parts by mass, with respect to 100 parts by mass of the monomers subjected to the polymerization step, from the viewpoint that polymerization heat is then appropriately removed, and thereby a polymerization temperature is easily controlled. In a case where a use amount of the hydrocarbon dispersion medium is 100 parts by mass or more, there is a tendency that it becomes easy to control a polymerization temperature. In a case where a use amount of the hydrocarbon dispersion medium is 1,000 parts by mass or less, there is a tendency that productivity of polymerization is improved, which is economic.

A chained water-absorbent resin having an internal cross-linking structure within the inside can be obtained by the self-cross-linking reaction that occurs during the polymerization. An internal cross-linking density of the inside may be adjusted, and liquid absorption characteristics of the chained water-absorbent resin may be controlled, by using an internal cross-linking agent during the polymerization. Examples of internal cross-linking agents to be used include polyols such as ethylene glycol, propylene glycol, trimethylolpropane, glycerin, polyoxyethylene glycol, polyoxypropylene glycol, and polyglycerin; di- or tri(meth)acrylic acid esters with the above polyols; unsaturated polyesters obtained by reacting the above polyols with unsaturated acids such as maleic acid and fumaric acid; bis(meth)acrylamides such as N,N'-methylenebis(meth)acrylamide; di- or tri(meth)acrylic acid esters obtained by reacting a polyepoxide with (meth)acrylic acid; carbamyl di(meth)acrylate esters obtained by reacting a polyisocyanate such as tolylene diisocyanate and hexamethylene diisocyanate with hydroxyethyl (meth)acrylate; compounds having two or more polymerizable unsaturated groups, such as allylated starch, allylated cellulose, diallyl phthalate, N,N',N"-triallylisocyanate, and divinylbenzene; polyglycidyl compounds such as (poly)ethylene glycol diglycidyl ether (where "(poly)" shall mean both with and without the "poly" prefix, and the same shall apply hereinafter), glycerin diglycidyl ether, glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; and compounds having two or more reactive functional groups, such as isocyanate compounds including, for example, 2,4-tolylene diisocyanate and hexamethylene diisocyanate. These cross-linking agents may be used alone or in combination of two or more kinds thereof.

An amount of the internal cross-linking agent added to the reaction system may be 0 mol% (that is, no internal cross-linking agent is used), but it is preferably more than 0 and 0.03 mole or less, is more preferably more than 0 and 0.01 mole or less, and is even more preferably more than 0 and 0.005 mole or less, per 1 mole of the monomer subjected to the polymerization step, from the viewpoint of ensuring that the obtained chained water-absorbent resin exhibits a sufficient water absorption amount.

The reverse phase suspension polymerization is performed in a water-in-oil system by mixing monomers subjected to the polymerization step, a water-soluble radical polymerization initiator, and if necessary, an internal cross-linking agent, a surfactant, a polymer-based dispersant, and a hydrocarbon dispersion medium, and heating the mixture under stirring. The order of addition of each of the components can be adjusted as appropriate. For example, it is suitable that the surfactant be mixed with the hydrocarbon dispersion medium in advance, each of the water-soluble radical polymerization initiator, the internal cross-linking agent, and the monomer be mixed, and mixed liquids obtained therefrom be mixed to start the polymerization. Furthermore, a multi-stage polymerization method in which the monomer aqueous solution is added in a plurality of times may be used. In a case where the multi-stage polymerization method is adopted, a monomer used in a first stage polymerization step preferably contains the monomer A and the monomer B. Furthermore, a monomer used in a second and subsequent polymerization steps preferably contains the monomer A, and more preferably contains only the monomer A. By using only the monomer A (that is, not using the monomer B) as the monomer to be subjected to the polymerization of the second and subsequent stages, it is possible to inhibit agglomeration of a hydrogel and to more efficiently obtain a chained water-absorbent resin. In a case where the multi-stage polymerization method is adopted, a use amount of the water-soluble radical polymerization initiator and the internal cross-linking agent in the polymerization step of each of the stages is preferably within the above-described range, based on the monomer subjected to the polymerization step of each of the stages. Furthermore, as necessary, a surfactant, a polymer-based dispersant, and a hydrocarbon dispersion medium may be added.

A temperature for the polymerization reaction varies depending on water-soluble radical polymerization initiators used, but it is preferably 20°C to 110°C, and is more preferably 40°C to 90°C, from the viewpoint that the polymerization is then promptly performed, which shortens a polymerization time, and thereby economic efficiency increases, and that polymerization heat is then easily removed, and thereby the reaction is smoothly performed. A reaction time is generally 0.1 to 4 hours. Completion of the polymerization reaction can be confirmed from, for example, stop of temperature rising in the reaction system. Accordingly, the chained water-absorbent resin is generally obtained in the state of a hydrogel.

<Drying step>

Subsequently, drying is performed to remove moisture from the obtained hydrogel. Examples of drying methods include a method (a) in which the hydrogel in a state of being dispersed in a hydrocarbon dispersion medium is subjected to azeotropic distillation by heating from the outside, and the hydrocarbon dispersion medium is refluxed to remove (dehydrate) moisture; a method (b) in which the hydrogel is taken out by decantation and dried under reduced pressure; and a method (c) in which the hydrogel is separated by filtration with a filter and dried under reduced pressure. Among them, it is preferable to use the method (a) to efficiently obtain the chained water-absorbent resin. Furthermore, by dehydrating the hydrocarbon dispersion medium by azeotropic distillation, a water content of the hydrogel is reduced to about 50% by mass, and thereby a chained water-absorbent resin can be efficiently obtained. When the hydrocarbon dispersion medium is first volatilized and dried in a state where a water content of the hydrogel is high, there is a high possibility that particles will be agglomerated and it will be difficult to obtain a chained water-absorbent resin.

The particle size of the particles constituting the chained water-absorbent resin described in the present embodiment can be controlled by, for example, an amount of a surfactant, an amount of a thickener, a stirring speed at the time of polymerization, and the like.

In the production of the chained water-absorbent resin according to the present embodiment, a surface portion of the hydrogel may be cross-linked (post-cross-linking) using a cross-linking agent in the drying step or any of subsequent steps. By such a treatment, a cross-linking density of the surface portion of the water-absorbent resin is increased, and thereby it is possible to obtain a chained water-absorbent resin suitable for sanitary material applications in which various performances such as a water absorption rate and a water-absorbing ability under pressure, and the like are improved. The post-cross-linking is preferably performed at a timing when the hydrogel has a specific water content. A timing of the post-cross-linking is preferably a time point at which a water content of the hydrogel is 10% to 60% by mass, is more preferably a time point at which a water content thereof is 20% to 55% by mass, and is even more preferably a time point at which a water content thereof is 30% to 50% by mass.

A water content (% by mass) of the hydrogel is calculated by the following formula.

$$\text{Water content} = [\text{Ww}/(\text{Ww} + \text{Ws})] \times 100$$

Ww: A moisture amount of a hydrogel obtained by adding a moisture amount used, as desired, upon mixing a post-cross-linking agent, and the like to an amount obtained by subtracting a moisture amount extracted to the outside by the drying process from a moisture amount contained in an aqueous liquid before polymerization in the all polymerization processes.

Ws: A solid fraction calculated from an amount of materials introduced, such as monomers, a cross-linking agent, and an initiator, each of which constitutes the hydrogel.

Examples of post-cross-linking agents for performing post-cross-linking include compounds having two or more reactive functional groups. Examples thereof include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and α-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis[N,N-di(β-hydroxyethyl)]adipamide. Among them, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are more preferable. These post-cross-linking agents may be used alone or in combination of two or more kinds thereof.

A use amount of the post-cross-linking agent varies depending on the type of post-cross-linking agent, and cannot be unconditionally determined, but it may be 0 mole (that is, a post-cross-linking agent is not used) with respect to 1 mole of the monomer (subjected to the polymerization step of the all stages in a case where the step is performed in multiple stages) subjected to the polymerization step. The use amount is more than 0 and 0.2 mol or less, is preferably more than 0 and 0.1 mol or less, and is more preferably more than 0 and 0.05 mol or less, from the viewpoint of sufficiently increasing a cross-linking density on the surface of the chained water-absorbent resin and increasing a gel strength of the chained water-absorbent resin.

In a case where the post-cross-linking reaction is carried out, a dried product of the chained water-absorbent resin can be obtained by distilling off water and the hydrocarbon dispersion medium by a known method.

The chained water-absorbent resin of the present disclosure can be used by, for example, adding gel stabilizers, metal chelating agents, lubricants, or the like. Examples of lubricants include aluminum oxide, titanium oxide, kaolin, talc, bentonite, amorphous silica, and the like. It is preferable to add amorphous silica because then production cost can be reduced and hindrance for a water absorption performance is low. A lower limit value of the amount of amorphous silica added to the water-absorbent resin is, for example, 0.05 parts by mass, and is preferably 0.1 parts by mass, with respect to a mass of the water-absorbent resin as a basis (100 parts by mass). Furthermore, an upper limit value thereof is, for example, 2 parts by mass, and is preferably 1 part by mass.

The chained water-absorbent resin according to the present embodiment can be made to have a desired particle size distribution at a timing obtained in the above-described production method, but their particle size distribution may be set to a predetermined particle size distribution by further performing operations such as adjustment of a particle size through classification with a sieve. Furthermore, in the above-mentioned production method, not only the chained water-absorbent resin but also the non-chain water-absorbent resin can be obtained. In this case, the chained water-absorbent resin can be selectively recovered by utilizing classification.

Since the chained water-absorbent resin of the present disclosure produced as described above is not bulkier than the hydrophilic fibers, when the chained water-absorbent resin is used as a substitute for the hydrophilic fibers, a thickness of the absorbent and the absorbent article can be made thinner as compared to those of the conventional absorbent and absorbent article. Furthermore, since the chained water-absorbent resin of the present disclosure is a water-absorbent resin, it has a high water retention amount by itself (on the other hand, the hydrophilic fibers have almost no water retention amount). Therefore, when the chained water-absorbent resin of the present disclosure is used as a substitute for the hydrophilic fibers, an amount of reversion from the absorbent and the absorbent article can be effectively reduced.

Furthermore, the chained water-absorbent resin of the present disclosure is capable of sufficiently diffusing a liquid. The reason for this is not clear, but the inventors of the present invention speculate as follows. It is thought that, in a conventional non-chain water-absorbent resin, when an absorbent is produced, each non-chain water-absorbent resin is easily dispersed in a non-contact and independent state with another non-chain water-absorbent resin, and this independent dispersion hinders diffusion of a liquid in the absorbent. On the other hand, it is thought that, since the chained water-absorbent resin of the present disclosure is elongated, each chained water-absorbent resin is easily dispersed in an absorbent in a state of being in contact with another chained water-absorbent resin. Therefore, a liquid can be sufficiently dispersed, and furthermore, the shape of the chained water-absorbent resin itself (the shape in which adjacent particles are continuously connected in a distinguishable manner) also contributes to the diffusion of the liquid.

Since the chained water-absorbent resin of the present disclosure produced as above has a softer tactile sensation than a conventional water-absorbent resin (non-chain water-absorbent resin), when the chained water-absorbent resin of the present disclosure is used as a substitute for the conventional water-absorbent resin, it is possible to make a sensation of the skin of the absorbent and the absorbent article more favorable as compared with a conventional absorbent and a conventional absorbent article. That is, it is possible to obtain an absorbent and an absorbent article having a soft sensation of the skin as compared with the conventional absorbent and the conventional absorbent article. Furthermore, the chained water-absorbent resin of the present disclosure is also a water-absorbent resin and has a sufficiently high water retention amount. Accordingly, even in a case where the chained water-absorbent resin of the present disclosure is used as a substitute for the non-chain water-absorbent resin, an absorption performance as the conventional absorbent and the conventional absorbent article can be sufficiently exhibited.

[Examples]

Hereinafter, the present disclosure will be described in more detail with reference to examples, but the present disclosure is not limited to these examples.

[Creation of water-absorbent resin according to Examples 1 to 5]

(Example 1)

A cylindrical round-bottomed separable flask was prepared, which had an inner diameter of 110 mm and a capacity of 2 L, and was equipped with a reflux condenser, a dropping funnel, a nitrogen gas introduction tube, and a stirring blade having four inclined paddle blades, each having a blade diameter of 50 mm, in a two-tier manner. 300 g of n-heptane as a hydrocarbon dispersion medium was put into this flask, and 0.37 g of a maleic anhydride-modified ethylene-propylene copolymer (trade name: Hi-WAX 1105A, manufactured by Mitsui Chemicals, Inc.) as a polymer-based dispersant, and 0.37 g of sucrose stearic acid ester (trade name: RYOTO Sugar Ester S-370, HLB value 3, manufactured by Mitsubishi-Chemical Foods Corporation) as a surfactant were added. The mixture was heated and dissolved while being stirred at 550 rpm, and thereafter, it was cooled to 55°C.

Meanwhile, 28 g (0.3 mole) of an aqueous solution of 80% by mass acrylic acid was put into an Erlenmeyer flask having a volume of 500 mL, and 31 g of an aqueous solution of 30% by mass sodium hydroxide was added dropwise while cooling the flask from the outside to perform neutralization. 0.028 g of hydroxyethyl cellulose (trade name: HEC AW-15F, manufactured by Sumitomo Seika Chemicals Co., Ltd.) as a thickener was added and dissolved. After dissolution of the thickener, 99 g (0.20 mole) of methoxypolyethylene glycol #400 acrylate, 0.046 g (0.17 mmol) of 2,2'-azobis (2-amidinopropane) dihydrochloride, which is an azo compound, as a polymerization initiator, 0.005 g (0.029 mmol) of ethylene glycol diglycidyl ether as an internal cross-linking agent, and 79.0 g of ion-exchanged water were added and dissolved. Thereby, a monomer aqueous solution was prepared. The above-mentioned methoxypolyethylene glycol #400 acrylate corresponds to a monomer in which n = 9 and R = $CH_3$ in a structural formula of the alkoxypolyalkylene glycol (meth)acrylate represented by General Formula (1).

The above-mentioned monomer aqueous solution was added to the above-mentioned separable flask into which the hydrocarbon dispersion medium, in which the polymer-based dispersant and the surfactant were dissolved, was injected. After sufficiently replacing the inside of the system with nitrogen while stirring the inside of the separable flask, the flask was immersed in a water bath at 70°C to raise the temperature. The polymerization reaction was carried out and completed, and thereby a hydrogel was obtained.

After the polymerization, a separable flask was immersed in an oil bath at 125°C to raise the temperature of the reaction solution, and the hydrogel was dehydrated while refluxing n-heptane by azeotropic distillation of n-heptane and water. Thereafter, 4.42 g (0.51 mmol) of an aqueous solution of 2% ethylene glycol diglycidyl ether was added to the hydrogel as a post-cross-linking agent. The mixture was maintained at 80°C for 2 hours, and then n-heptane was distilled off and dried. The obtained polymer was passed through sieves having an opening of 850 $\mu$m and 250 $\mu$m, and 40.5 g of the polymer that passed through 850 $\mu$m remained on the 250 $\mu$m sieve was recovered. Thereafter, an amount of amorphous silica (trade name: CARPLEX #80, manufactured by Evonik Degussa Japan Co., Ltd.) corresponding to 0.2% by mass of the recovered polymer was added to and mixed with the recovered polymer (40.5 g), and evaluation of the chained water-absorbent resin according to Example 1 was performed.

(Example 2)

28.2 g of a polymer was recovered by performing the same operation as in Example 1 except that, in Example 1, polymerization was carried out using 135 g (0.20 mole) of methoxypolyethylene glycol #550 acrylate instead of 99 g (0.20 mole) of methoxypolyethylene glycol #400 acrylate, and using, as a polymerization initiator, 0.046 g (0.17 mmol) of potassium persulfate instead of 0.046 g (0.17 mmol) of 2,2'-azobis (2-amidinopropane) dihydrochloride as an azo compound. Amorphous silica in an amount corresponding to 0.2% by mass of the recovered polymer was added to and mixed with a polymer (28.2 g) recovered in the same manner as in Example 1, and a chained water-absorbent resin of Example 2 was obtained. Thereafter, the chained water-absorbent resin of Example 2 was evaluated according to an evaluation method to be described later.

The above-mentioned methoxypolyethylene glycol #550 acrylate corresponds to a monomer in which n = 13 and R = $CH_3$ in a structural formula of the alkoxypolyalkylene glycol (meth)acrylate represented by General Formula (1).

(Example 3)

35.3 g of the polymer was recovered by performing the same operation as in Example 1 except that, in Example 1, polymerization was performed using 0.37 g of polyglycerate (trade name: Polyglycerol Ester B-100D, HLB value 3, manufactured by Mitsubishi-Chemical Foods Corporation) instead of 0.37 g of sucrose stearic acid ester as a surfactant. Amorphous silica in an amount corresponding to 0.2% by mass of the recovered polymer was added to and mixed with a polymer (35.3 g) recovered in the same manner as in Example 1, and a chained water-absorbent resin of Example 3 was obtained. Thereafter, the chained water-absorbent resin of Example 3 was evaluated according to an evaluation method to be described later.

[Example 4]

300 g of exole heptane as a hydrocarbon dispersion medium was put into the same reaction device as in Example 1, and 0.37 g of a maleic anhydride-modified ethylene-propylene copolymer (trade name: Hi-WAX 1105A, manufactured by Mitsui Chemicals, Inc.) as a polymer-based dispersant, and 0.37 g of sucrose stearic acid ester (trade name: RYOTO Sugar Ester S-370, HLB value 3, manufactured by Mitsubishi-Chemical Foods Corporation) as a surfactant were added. The mixture was heated and dissolved while being stirred at 400 rpm, and thereafter, it was cooled to 55°C.

Meanwhile, 28 g (0.3 mole) of an aqueous solution of 80% by mass acrylic acid was put into an Erlenmeyer flask having a volume of 500 mL, and 31 g of an aqueous solution of 30% by mass sodium hydroxide was added dropwise while cooling the flask from the outside to perform neutralization. 0.022 g of hydroxyethyl cellulose (trade name: HEC AW-15F, manufactured by Sumitomo Seika Chemicals Co., Ltd.) as a thickener was added and dissolved. After dissolution of the thickener, 99 g (0.20 mole) of methoxypolyethylene glycol #400 acrylate, 0.046 g (0.17 mmol) of 2,2'-azobis (2-amidinopropane) dihydrochloride, which is an azo compound, as a polymerization initiator, 0.005 g (0.029 mmol) of ethylene glycol diglycidyl ether as an internal cross-linking agent, and 172.7 g of ion-exchanged water were added and dissolved. Thereby, a monomer aqueous solution was prepared.

The above-mentioned monomer aqueous solution was added to the above-mentioned separable flask into which the hydrocarbon dispersion medium, in which the polymer-based dispersant and the surfactant were dissolved, was injected. After sufficiently replacing the inside of the system with nitrogen while stirring the inside of the separable flask, the flask was immersed in a water bath at 70°C to raise the temperature. The polymerization reaction was carried out and completed, and thereby a hydrogel was obtained.

Subsequent operations were the same as in Example 1, and 44.6 g of a polymer was recovered. Amorphous silica in an amount corresponding to 0.2% by mass of the recovered polymer was added to and mixed with a polymer (44.6 g) recovered in the same manner as in Example 1, and a chained water-absorbent resin of Example 4 was obtained. Thereafter, the chained water-absorbent resin of Example 4 was evaluated according to an evaluation method to be described later.

(Example 5)

416 g of n-heptane as a hydrocarbon dispersion medium was put into the same reaction device as in Example 1, and 0.37 g of a maleic anhydride-modified ethylene-propylene copolymer (trade name: Hi-WAX 1105A, manufactured by Mitsui Chemicals, Inc.) as a polymer-based dispersant, and 0.37 g of sucrose stearic acid ester (trade name: RYOTO Sugar Ester S-370, HLB value 3, manufactured by Mitsubishi-Chemical Foods Corporation) as a surfactant were added. The mixture was heated and dissolved while being stirred at 550 rpm, and thereafter, it was cooled to 55°C.

Meanwhile, 28 g (0.3 mole) of an aqueous solution of 80% by mass acrylic acid was put into an Erlenmeyer flask having a volume of 500 mL, and 31 g of an aqueous solution of 30% by mass sodium hydroxide was added dropwise while cooling the flask from the outside to perform neutralization. 0.028 g of hydroxyethyl cellulose (trade name: HEC AW-15F, manufactured by Sumitomo Seika Chemicals Co., Ltd.) as a thickener was added and dissolved. After dissolution of the thickener, 99 g (0.20 mole) of methoxypolyethylene glycol #400 acrylate, 0.046 g (0.17 mmol) of 2,2'-azobis (2-amidinopropane) dihydrochloride, which is an azo compound, as a polymerization initiator, 0.005 g (0.029 mmol) of ethylene glycol diglycidyl ether as an internal cross-linking agent, and 172.7 g of ion-exchanged water were added and dissolved. Thereby, a monomer aqueous solution was prepared.

The above-mentioned monomer aqueous solution was added to the above-mentioned separable flask into which the hydrocarbon dispersion medium, in which the polymer-based dispersant and the surfactant were dissolved, was injected. After sufficiently replacing the inside of the system with nitrogen while stirring the inside of the separable flask, the flask was immersed in a water bath at 70°C to raise the temperature. The polymerization reaction was carried out and completed, and thereby a hydrogel was obtained.

Subsequent operations were the same as in Example 1, and 36.9 g of a polymer was recovered. Amorphous silica in an amount corresponding to 0.2% by mass of the recovered polymer was added to and mixed with a polymer (36.9 g) recovered in the same manner as in Example 1, and a chained water-absorbent resin of Example 5 was obtained. Thereafter, the chained water-absorbent resin of Example 5 was evaluated according to an evaluation method to be described later.

(Example 6)

A cylindrical round-bottomed separable flask was prepared, which had an inner diameter of 110 mm and a capacity of 2 L, and was equipped with a reflux condenser, a dropping funnel, a nitrogen gas introduction tube, and a stirring blade having four inclined paddle blades, each having a blade diameter of 50 mm, in a two-tier manner. 300 g of n-heptane as a hydrocarbon dispersion medium was put into this flask, and 0.37 g of a maleic anhydride-modified ethylene-propylene copolymer (trade name: Hi-WAX 1105A, manufactured by Mitsui Chemicals, Inc.) as a polymer-based dispersant, and 0.37 g of sucrose stearic acid ester (trade name: RYOTO Sugar Ester S-370, HLB value 3, manufactured by Mitsubishi-Chemical Foods Corporation) as a surfactant were added. The mixture was heated and dissolved while being stirred at 550 rpm, and thereafter, it was cooled to 55°C.

Meanwhile, 28 g (0.3 mole) of an aqueous solution of 80% by mass acrylic acid was put into an Erlenmeyer flask having a volume of 500 mL, and 31 g of an aqueous solution of 30% by mass sodium hydroxide was added dropwise while cooling the flask from the outside to perform neutralization. 0.028 g of hydroxyethyl cellulose (trade name: HEC AW-15F, manufactured by Sumitomo Seika Chemicals Co., Ltd.) as a thickener was added and dissolved. After dissolution of the thickener, 99 g (0.20 mole) of methoxypolyethylene glycol #400 acrylate, 0.046 g (0.17 mmol) of 2,2'-azobis (2-amidinopropane) dihydrochloride, which is an azo compound, as a polymerization initiator, 0.005 g (0.029 mmol) of ethylene glycol diglycidyl ether as an internal cross-linking agent, and 79.0 g of ion-exchanged water were added and dissolved. Thereby, a first stage monomer aqueous solution of was prepared.

The above-mentioned first stage monomer aqueous solution was added to the above-mentioned separable flask into which the hydrocarbon dispersion medium in which the polymer-based dispersant and the surfactant were dissolved was injected. After sufficiently replacing the inside of the system with nitrogen while stirring the inside of the separable flask, the flask was immersed in a water bath at 70°C to raise the temperature. The polymerization reaction was carried out and completed, and thereby a first stage hydrogel was obtained.

Meanwhile, 64.4 g (0.71 mole) of an aqueous solution of 80% by mass acrylic acid was put into a 500 mL Erlenmeyer flask, and 71.6 g of an aqueous solution of 30% by mass sodium hydroxide was added dropwise while cooling the flask from the outside to perform 75 mol% neutralization. Into the flask, 0.065 g (0.238 mmol) of 2,2'-azobis (2-amidinopropane) dihydrochloride, which is an azo compound, as a polymerization initiator, 0.007 g (0.040 mmol) of ethylene glycol diglycidyl ether as an internal cross-linking agent, and 5.7 g of ion-exchanged water were added and dissolved. Thereby, a second stage monomer aqueous solution was prepared.

After cooling the first stage hydrogel to 25°C, the entire amount of the second stage monomer aqueous solution was added to the first stage hydrogel, and a temperature was maintained at 25°C for 30 minutes while replacing the inside of the system with nitrogen. Thereafter, the flask was again immersed in a water bath at 70°C to raise the temperature. The polymerization reaction was carried out and completed, and thereby a second stage hydrogel was obtained.

After the polymerization, a separable flask was immersed in an oil bath at 125°C to raise the temperature of the reaction solution, and the hydrogel was dehydrated while refluxing n-heptane by azeotropic distillation of n-heptane and water. Thereafter, n-heptane was distilled off and dried. The obtained polymer was passed through sieves having an opening of 850 $\mu$m and 250 $\mu$m, and 58.5 g of the polymer that passed through 850 $\mu$m remained on the 250 $\mu$m sieve was recovered. Thereafter, an amount of amorphous silica (trade name: CARPLEX #80, manufactured by Evonik Degussa Japan Co., Ltd.) corresponding to 0.2% by mass of the recovered polymer was added to and mixed with the recovered polymer (58.5 g), and a chained water-absorbent resin according to Example 6 was obtained. Thereafter, the chained water-absorbent resin of Example 6 was evaluated according to an evaluation method to be described later.

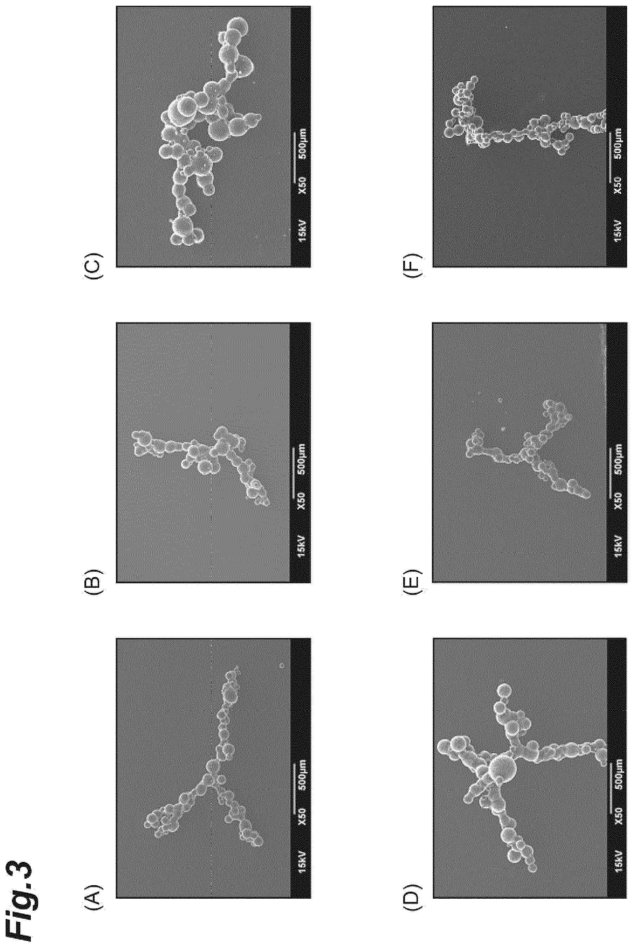

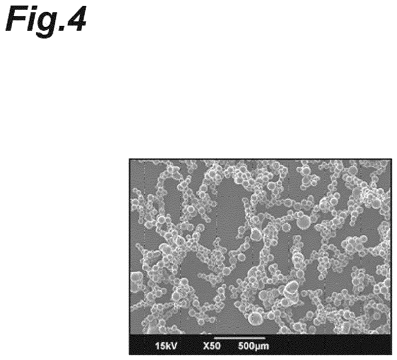

SEM images of one chained water-absorbent resin contained in the water-absorbent resins obtained in Examples 1 to 6 are shown in Figs. 3(A) to 3(F). The SEM image of one chained water-absorbent resin contained in the water-absorbent resin obtained in Example 1 corresponds to Fig. 3(A). Similarly, Example 2 corresponds to Fig. 3(B), Example 3 corresponds to Fig. 3(C), Example 4 corresponds to Fig. 3(D), Example 5 corresponds to Fig. 3(E), and Example 6 corresponds to Fig. 3(F). Furthermore, Fig. 4 shows an SEM image of a plurality of water-absorbent resins randomly acquired from the water-absorbent resins obtained in Example 1.

[Evaluation of water-absorbent resin]

(Water retention amount for physiological saline)

500 g of an aqueous solution of 0.9% by mass sodium chloride (physiological saline) was weighed into a beaker having a volume of 500 mL, and while stirring the solution using a magnetic stirrer bar (8 mm$\varphi$ $\times$ 30 mm, without a ring) at 600 r/min, 2.0 g of each of the water-absorbent resins of Examples 1 to 5 was dispersed therein such that a lump (clump) was not generated. The mixture was left to stand for 30 minutes in a stirred state to cause the water-absorbent resin to swell sufficiently. Thereafter, the mixture was poured into a cotton bag (cotton broadcloth No. 60, width 100 mm x length 200 mm), and an upper part of the cotton bag was tied with a rubber band. The cotton bag after an elapse of 30 minutes was dehydrated for 1 minute using a dehydrator (manufactured by KOKUSAN Co., Ltd., product number: H-122) which had been set at a centrifugal force of 167 G, and a mass Wa (g) of the dehydrated cotton bag containing the swollen gel was measured. By performing the same operation without addition of the water-absorbent resin, a mass Wb (g) of an empty cotton bag upon moisturizing was measured, and a water retention amount of the water-absorbent resin of each of the examples was calculated by the following formula.

$$\text{Water retention amount for physiological saline (g/g)} = [\text{Wa} - \text{Wb}] \text{ (g)/mass of water-absorbent resin (g)}$$

(Water absorption rate for physiological saline)

A water absorption rate for physiological saline was measured in a room adjusted to 25°C ± 1°C. 50 ± 0.1 g of physiological saline that has been put into a beaker having a volume of 100 mL was adjusted to a temperature of 25°C ± 0.2°C in a thermostatic water bath, and then stirred at 600 rpm with a magnetic stirrer bar (8 mm$\varphi$ × 30 mm, without a ring) to generate a vortex. 2.0 ± 0.002 g of the water-absorbent resin was added into the above physiological saline at once, and a time (sec) until the vortex disappeared and the liquid surface became flat after the addition of the water-absorbent resin was measured. The time was taken as a water absorption rate of the water-absorbent resin for physiological saline.

(Water-absorbing ability under pressure)

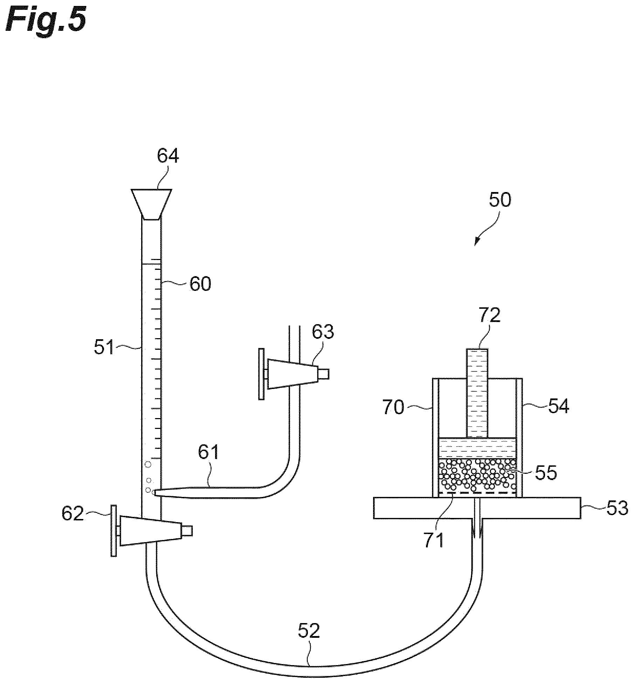

Fig. 5 shows a schematic view of a measurement device used for measuring a water-absorbing ability under pressure of the water-absorbent resin. The measurement was performed using a measurement device 50 shown in Fig. 5. The measurement device 50 shown in Fig. 5 is composed of a burette unit 51, a conduit 52, a measurement table 53, and a measuring unit 54 placed on the measurement table 53. In the burette unit 51, a rubber stopper 64 is connected to the upper part of a burette 60, and an air introduction tube 61 and a cock 62 are respectively connected to a lower part. The air introduction tube 61 has a cock 63 at its distal end. The conduit 52 is attached between the burette unit 51 and the measurement table 53. An inner diameter of the conduit 52 is 6 mm. A hole with a diameter of 2 mm is present in a central portion of the measurement table 53, and the conduit 52 is connected to the hole. The measuring unit 54 includes a cylinder 70 (made of plexiglass), a nylon mesh 71 adhered to a bottom portion of this cylinder 70, and a weight 72. An inner diameter of the cylinder 70 is 20 mm. An opening of the nylon mesh 71 is 75 $\mu$m (200 mesh). Then, at the time of measurement, a water absorption agent 55 (water-absorbent resin that is a measurement target) is uniformly sprinkled on the nylon mesh 71. The weight 72 has a diameter of 19 mm and a mass of 60 g. The weight 72 is placed on the water absorption agent 55 so that a load of 2.07 kPa can be applied to the water absorption agent 55.

After 0.100 g of the water-absorbent resin as a measurement target was put in the acrylic resin cylinder 70 of the measurement device 50 shown in Fig. 5, the weight 72 was placed thereon to start the measurement. The same volume of air as the physiological saline absorbed by the water-absorbent resin was quickly and smoothly supplied to the inside of the burette through the air introduction tube, and therefore a reduced amount in water level of the physiological saline inside the burette was an amount of the physiological saline absorbed by the water-absorbent resin. A scale of the burette was engraved from 0 ml in increments of 0.5 ml, from the top to bottom direction. A scale $V_A$ of the burette before the start of water absorption and a scale $V_B$ of the burette 60 minutes after the start of water absorption were read as water levels of the physiological saline, and a water-absorbing ability under pressure was calculated from the following formula.

$$\text{Water-absorbing ability under pressure [ml/g]} = (V_B - V_A)/0.1$$

Table 1 shows results of the water-absorbent resins according to Examples 1 to 6, which are related to a water retention amount for physiological saline, a water absorption rate for physiological saline, and water-absorbing ability under pressure.

[Table 1]

| | Water retention amount for physiological saline | Water absorption rate for physiological saline | Water-absorbing ability under pressure |
|---|---|---|---|
| Example 1 | 30 g/g | 33 seconds | 7.6 ml/g |
| Example 2 | 28 g/g | 38 seconds | 6.9 ml/g |
| Example 3 | 30 g/g | 59 seconds | 6.6 ml/g |
| Example 4 | 30 g/g | 44 seconds | 7.8 ml/g |
| Example 5 | 42 g/g | 42 seconds | 6.3 ml/g |
| Example 6 | 28 g/g | 34 seconds | 6.5 ml/g |

[Evaluation 1 of absorbent and absorbent article]

(1) Preparation of test solution (artificial urine)

An appropriate amount of distilled water was put into a container having a volume of 10 L, and 60 g of sodium chloride, 1.8 g of a calcium chloride dihydrate, and 3.6 g of a magnesium chloride hexahydrate were added and dissolved therein. Then, 0.15 g of polyoxyethylene nonylphenyl ether was added thereto, and distilled water was further added so that a total mass became 6,000 g. Furthermore, the mixed solution was colored with a small amount of Blue No. 1, and thereby a test solution was prepared.

(2) Production of absorbent and absorbent article

A non-chain water-absorbent resin (trade name: AQUA KEEP SA60S, manufactured by Sumitomo Seika Chemicals Co., Ltd.) composed of particles having a tuft-like shape of grapes, in which single particles are agglomerated, was prepared. The non-chain water-absorbent resin contains 0.1 part by mass of amorphous silica with respect to 100 parts by mass of the water-absorbent resin.

The non-chain water-absorbent resin, the chained water-absorbent resin according to Example 1 or Example 5, and/or crushed pulp (Reiflock manufactured by Leonia) were uniformly mixed by air papermaking so that a total weight was 20 g in composition ratios shown in Table 2, and thereby a sheet-shaped absorption core having a size of 40 cm × 12 cm was produced.

Next, after spraying 0.6 g of ion-exchanged water on the entire absorbent core by spraying, an absorbent was produced by applying a load of 141 kPa to the entire absorbent core for 30 seconds and pressing it in a state of sandwiching the absorbent core between two sheets of tissue paper (core wraps) having the same size as the absorbent core and having a basis weight of 16 g/m². Furthermore, a polyethylene air-through type porous liquid permeable sheet having a basis weight of 22 g/m², which is the same size as the absorbent, was disposed on the upper surface of the absorbent to prepare an absorbent article.

The composition ratios of the water-absorbent resin, pulp, and chained water-absorbent resin were changed as shown in Table 2 to produce absorbent articles according to Examples A to D and Comparative Example A. The water-absorbent resins used in the production of Examples A to D are those according to Example 1 or 5.

(3) Evaluation of thickness

The thickness of the absorbent article was obtained by measuring a central portion of the absorbent article with a thickness measurement device (model number: J-B, manufactured by OZAKI MFG. CO., LTD.).

(4) Amount of reversion

The absorbent article was placed on a horizontal table. A measuring instrument equipped with a liquid injection cylinder having an inner diameter of 3 cm was placed on the center of the absorbent article, and 50 mL of artificial urine was injected into the cylinder at once (first time). Next, the cylinder was removed, the absorbent article was left to stand as it is, and the same operation was performed using a measuring instrument at the same position as in the first time 30 minutes (second time) and 60 minutes (third time) after the start of the injection of the test solution of the first time. After a lapse of 60 minutes from the injection of a third time, 40 sheets of 10 cm square filter paper whose mass (Wc (g)) was measured in advance were placed near the injection position of the test solution on the absorbent article, and a weight having a bottom surface of 10 cm × 10 cm and a mass of 5 kg was placed thereon. After loading for 5 minutes, a mass of the filter paper (Wd (g)) was measured, and the increased mass was defined as an amount of reversion (g). It can be said that as an amount of reversion becomes smaller, an absorbent article is more preferable.

$$\text{Amount of reversion (g)} = \text{Wd} - \text{Wc}$$

(5) Diffusion length

Within 5 minutes after measuring the amount of reversion, a spread dimension (cm) in a longitudinal direction of the absorbent article permeated with the test solution was measured. A diffusion length was measured at the center, the left end, and the right end in a lateral direction, and an average thereof was taken as a numerical value. Numerical values after the decimal point were rounded off.

Table 2 shows the composition ratios of the absorbent constituting the absorbent articles according to Examples A to D and Comparative Example A, and evaluation results of a thickness, an amount of reversion, and a diffusion length. As shown in Table 2, the absorbent articles according to Examples A to D could be made significantly thinner than the absorbent article according to Comparative Example A, and although the absorbent articles according to Examples A to D had a diffusion length inferior than that of Comparative Example A, it was confirmed that an amount of reversion was significantly reduced, and it was confirmed that the absorbent articles according to Examples A to D had sufficient performance as an absorbent article.

As shown in Table 1, the chained water-absorbent resin of Example 1 or 5 and the chained water-absorbent resins of Examples 2 to 4 and 6 have the same level of water-absorbing ability (a water retention amount for physiological saline, a water absorption rate for physiological saline, and water-absorbing ability under pressure). Therefore, even when the chained water-absorbent resin of Examples 1 or 5 used for the absorbent articles of Examples A to D is used as a substitute for the chained water-absorbent resins of Examples 2 to 4 and 6, it is estimated that the same results as in Table 2 can be obtained.

[Table 2]

| | Type of chained water-absorbent resin | Configuration of absorption core (% by mass) | | | Thickness (mm) | Amount of reversion (g) | Diffusion length (mm) |
|---|---|---|---|---|---|---|---|
| | | Non-chain water-absorbent resin | Crushed pulp | Chained water-absorbent resin | | | |
| Example A | Example 1 | 50 | 25 | 25 | 2.4 | 16 | 20 |
| Example B | Example 1 | 50 | 0 | 50 | 1.8 | 10 | 19 |
| Example C | Example 1 | 25 | 25 | 50 | 2.8 | 12 | 26 |
| Example D | Example 5 | 50 | 0 | 50 | 1.9 | 5 | 20 |
| Comparative Example A | - | 50 | 50 | 0 | 4.0 | 33 | 27 |

[Evaluation 2 of absorbent and absorbent article]

(1) Production of absorbent and absorbent article

A non-chain water-absorbent resin (trade name: AQUA KEEP SA60S, manufactured by Sumitomo Seika Chemicals Co., Ltd.) composed of particles having a tuft-like shape of grapes, in which single particles are agglomerated, crushed pulp (Reiflock manufactured by Leonia), and the water-absorbent resin of Example 1 (chained water-absorbent resin) were prepared. These were uniformly mixed by air papermaking so that a total weight was 8 g in composition ratios shown in Table 3. Thereafter, a tissue paper having a size of 40 cm × 12 cm (basis weight 16 g/m$^2$) was uniformly sprayed to form an absorbent core. Furthermore, a tissue paper having a size of 40 cm × 12 cm (basis weight 16 g/m$^2$) was superimposed. Two sheets of the upper and lower tissue paper correspond to core wraps.

Next, after spraying 0.6 g of ion-exchanged water from above by spraying, an absorbent was produced by applying a load of 141 kPa to the entire absorption core for 30 seconds and pressing it.

An absorbent having a size of 20 cm × 6 cm was cut out from the center of the produced absorbent, and this was used as absorbents for test according to the following examples and a comparative example.

The composition ratios of the water-absorbent resin, pulp, and chained water-absorbent resin were changed as shown in Table 3 to produce absorbents for test according to Examples E to G and Comparative Example B.

(2) Evaluation of thickness

The thickness of the absorbent for test was obtained by measuring a central portion of the absorbent for test with a thickness measurement device (model number: J-B, manufactured by OZAKI MFG. CO., LTD.).

(3) Evaluation of deviation of friction coefficient (roughness) and surface roughness

For the purpose of evaluating characteristics related to a sensation of the skin, a deviation of friction coefficient (MMD) and a surface roughness (SMD) of the absorbent for test were measured using an automated surface tester KES-FB4-AUTO-A (manufactured by Kato Tech Co., Ltd.).

Specifically, the absorbent for test was placed on a test table of the device and fixed using a rod-shaped "Chuck" or tape. A load of 50 g was applied to a 10 mm × 10 mm terminal for friction wound with a wire having a diameter of 0.5 mm, and surface characteristics were measured by sliding the surface of the absorbent for test at a speed of 1 mm/sec. From measurement results, a deviation of friction coefficient (MMD) was obtained. The deviation of friction coefficient (MMD) is an index of roughness, and indicates more roughness as a value becomes larger.

At the same time as measuring the friction characteristics, surface characteristics were measured by applying a load of 10 g to a terminal for roughness made of a single wire with a width of 5 mm and sliding the surface of the absorbent for test at a speed of 1 mm/sec. Thereby, a surface roughness (SMD) was obtained from the result. The surface roughness (SMD) is an index related to unevenness of a surface, and indicates that unevenness of a surface is severe as a value becomes larger.

Table 3 shows composition ratios of the absorbent cores constituting the absorbents for test according to Examples E to G and Comparative Example B, and evaluation results of a thickness of the absorbents for test, a deviation of friction coefficient (MMD), and a surface roughness (SMD). As shown in Table 3, the absorbents for test according to Examples E to G have favorable characteristics relating to a sensation of the skin as compared to the absorbent for test according to Comparative Example B. In particular, it was confirmed that the absorbents for test according to Examples E to G have a small value of a surface roughness (SMD).

[Table 3]

| | Configuration of absorption core (% by mass) | | | Thickness (mm) | Characteristics relating to sensation of skin | |
|---|---|---|---|---|---|---|
| | Non-chain water-absorbent resin | Crushed pulp | Chained water-absorbent resin | | MMD | SMD |
| Comparative Example B | 50 | 50 | 0 | 1.3 | 0.019 | 5.4 |
| Example E | 37.5 | 50 | 12.5 | 1.1 | 0.019 | 4.3 |
| Example F | 25 | 50 | 25 | 1.2 | 0.018 | 1.9 |
| Example G | 0 | 50 | 50 | 1.0 | 0.0048 | 1.8 |

[Additional Notes]

There is room for improvement in absorbents formed using the conventional water-absorbent resin and absorbent articles formed using this absorbent from the viewpoint of a sensation of the skin. That is, because the surface of a conventional water-absorbent resin itself is hard, an unevenness of the water-absorbent resin is likely to be felt when pulp of an absorbent is reduced to make the absorbent thinner.

With the foregoing in view, it is an object of the present disclosure to provide an absorbent having a pleasant sensation of the skin, and an absorbent article containing the absorbent.

The inventors of the present invention have found that, in a case where a water-absorbent resin used for an absorbent has a specific shape, a sensation of the skin becomes pleasant when the absorbent is formed by combining this water-absorbent resin with pulp.

That is, an absorbent according to one aspect of the present disclosure contains a water-absorbent resin having a shape in which a plurality of particles having a substantially spherical shape is connected in a chain shape, and hydrophilic fibers.

Because the water-absorbent resin having a shape in which a plurality of particles having a substantially spherical shape is connected in a chain shape is softer than a conventional water-absorbent resin, a sensation of the skin becomes favorable according to the absorbent formed using this water-absorbent resin. In the present disclosure, the sentence "a sensation of the skin is pleasant (is favorable)" refers to a state in which both deviation of friction coefficient (MMD) and surface roughness (SMD), which represent friction characteristics, show small values, and a state in which a smooth tactile sensation can be obtained.

Furthermore, the water-absorbent resin may be a copolymer of two or more monomers.

Furthermore, an absorbent article according to one aspect of the present disclosure contains the above-described absorbent.

The above-described absorbent article contains the absorbent containing the above-described water-absorbent resin having the shape in which a plurality of particles having a substantially spherical shape is connected in a chain shape. Accordingly, a sensation of the skin becomes favorable also according to the absorbent article.

**Reference Signs List**

1       absorbent article
2       absorbent
21      water-absorbent resin
211     chained water-absorbent resin
40      particles.


**Claims**

1. A water-absorbent resin,
   wherein the water-absorbent resin has a shape in which a plurality of particles having a substantially spherical shape is connected in a chain shape.

2. The water-absorbent resin according to claim 1,
   wherein the water-absorbent resin is a copolymer of two or more monomers.

3. An absorbent comprising the water-absorbent resin according to claim 1 or 2.

4. An absorbent comprising:

   a water-absorbent resin having a shape in which a plurality of particles having a substantially spherical shape is connected in a chain shape; and
   hydrophilic fibers.

5. The absorbent according to claim 4,
   wherein the water-absorbent resin is a copolymer of two or more monomers.

6. An absorbent article comprising the absorbent according to claim 4 or 5.

7. A method for producing a water-absorbent resin, the method comprising:

   copolymerizing a monomer A and a monomer B by a reverse phase suspension polymerization method; and
   drying a hydrogel obtained in the copolymerizing,
   wherein the monomer A contains at least one of (meth)acrylic acid and a neutralized product thereof,
   the monomer B contains alkoxypolyalkylene glycol (meth)acrylate, and
   in the copolymerizing, a ratio $(\alpha/\beta)$ of a molar amount $(\alpha)$ of the monomer A to be provided for the copolymerization to a molar amount $(\beta)$ of the monomer B to be provided for the copolymerization is 1/4 to 4.

8. The method for producing a water-absorbent resin according to claim 7,
   wherein, in the copolymerizing, at least one of sucrose fatty acid ester and polyglycerin fatty acid ester is added to a reaction system as a surfactant.

9. The method for producing a water-absorbent resin according to claim 7 or 8,
   wherein, in the copolymerizing, polymerization is performed in the presence of a polymer-based dispersant.

10. The method for producing a water-absorbent resin according to any one of claims 7 to 9,
    wherein, in the drying, the hydrogel is dehydrated by azeotropic distillation of a hydrocarbon dispersion medium until a water content reaches 50% by mass or less.

Fig.1

# *Fig.2*

(A)

(B)

*Fig.3*

# Fig.4

# Fig.5

<div align="center">**INTERNATIONAL SEARCH REPORT**</div>

| | International application No. |
|---|---|
| | PCT/JP2019/047068 |

**A. CLASSIFICATION OF SUBJECT MATTER**
Int.Cl. C08F6/24(2006.01)i, C08F220/06(2006.01)i, C08F220/28(2006.01)i,
C08F2/32(2006.01)i, B01J20/26(2006.01)i, B01J20/28(2006.01)i,
B01J20/30(2006.01)i
FI: C08F2/32, B01J20/26D, B01J20/28A, B01J20/30, C08F6/24, C08F220/06,
C08F220/28

According to International Patent Classification (IPC) or to both national classification and IPC

**B. FIELDS SEARCHED**

Minimum documentation searched (classification system followed by classification symbols)
Int.Cl. C08F6/24, C08F220/06, C08F220/28, C08F2/32, B01J20/26, B01J20/28,
B01J20/30

Documentation searched other than minimum documentation to the extent that such documents are included in the fields searched
| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2016-28117 A (SUMITOMO SEIKA CHEMICALS CO., LTD.) 25.02.2016 (2016-02-25), claims, paragraphs [0026], [0089], example 1 | 1–6 |
| X | JP 2007-197873 A (MITSUBISHI CHEMICAL CORPORATION) 09.08.2007 (2007-08-09), claims, paragraph [0035] | 1–6 |
| A | JP 2005-97593 A (NIPPON SHOKUBAI CO., LTD.) 14.04.2005 (2005-04-14), entire text | 1–10 |
| A | JP 2005-15787 A (NIPPON SHOKUBAI CO., LTD.) 20.01.2005 (2005-01-20), entire text | 1–10 |
| A | JP 7-102006 A (NIPPON CHEMICAL INDUSTRIAL CO., LTD.) 18.04.1995 (1995-04-18), entire text | 1–10 |

☐ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 19.02.2020 | 03.03.2020 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

# EP 3 901 182 A1

| INTERNATIONAL SEARCH REPORT | International application No. |
| Information on patent family members | PCT/JP2019/047068 |

| | | | |
|---|---|---|---|
| JP 2016-28117 A | 25.02.2016 | US 2017/0107313 A1<br>claims, paragraphs [0028], [0081],<br>example 1<br>WO 2016/006133 A1<br>EP 2993191 A1 | |
| JP 2007-197873 A | 09.08.2007 | (Family: none) | |
| JP 2005-97593 A | 14.04.2005 | US 2005/0049379 A1<br>entire text<br>EP 1512417 A1 | |
| JP 2005-15787 A | 20.01.2005 | US 2004/0249120 A1<br>entire text | |
| JP 7-102006 A | 18.04.1995 | (Family: none) | |

Form PCT/ISA/210 (patent family annex) (January 2015)

28

**REFERENCES CITED IN THE DESCRIPTION**

**Patent documents cited in the description**

- JP 2009084472 A